# EUROPEAN PATENT APPLICATION

(11) **EP 2 873 363 A1**
(43) Date of publication of application: **20.05.2015**
(21) Application number: 13816943.8
(22) Date of filing: 11.07.2013
(51) Int. Cl.: A61B 3/024

(54) **DIOPSIMETER**

(30) Priority: 13.07.2012 JP 2012157400
(71) Applicant: Kowa Company, Ltd., Nagoya-shi, Aichi-ken 460-8625 (JP)
(72) Inventor: HARA, Takuya, Hamamatsu-shi Shizuoka 431-2103 (JP)
(74) Representative: Nobbe, Matthias
(86) International application number: PCT/JP2013/068958
(87) International publication number: WO 2014/010664

(57) **Abstract**

In a perimeter 2 having means 19 for setting a measurement coordinate system CS for perimetry, a means 13 for detecting a position M1 of a blind spot of a subject eye based upon the set measurement coordinate system CS, a means 19 for measuring a visual field of the same subject eye 22a on the basis of the respectively set measurement coordinate systems CS_{F} and CS_{N} at the first point of time and a subsequent second point of time after the first point of time, such a perimeter has a means 11 for storing the position (r, *θ*) of the blind spot that is detected at the first point of time in a memory 15, a means 16 for detecting the position (r', *θ*') of the blind spot of the subject eye at the perimetry in the second point of time of perimetry and computing amount of deviation (α) with the position (r, *θ*) of the blind spot at the first point of time, and a means 17 for conducting such an operation that the new measurement coordinate system CS_{N} is moved on the basis of the amount of deviation (α) so as to correspond with the measurement coordinate system CS_{F} at the first point of time, and conducts the perimetry on the basis of the measurement coordinate system CS_{N} that correspond with the measurement coordinate system CS_{F} at the first point of time.

## Description

### TECHNICAL FIELD

The invention relates to a perimeter that is possible to examine subject eyes at the same position each time at a time of perimetry.

### BACKGROUND ART

Perimetry is conducted by testing sensitivity of light stimulation provided on each retina part of an examinee in such a way that targets having various kinds of sizes and brightness are presented to the examinee when the examinee fixates a fixation point that is set at a center of a visual field dome and the examinee responds if the examinee perceives the target.

Generally, examination algorithm for determining a position of a blind spot is built in the perimeter (see below-mentioned Patent related document 1), and at a time of perimetry, the examinee is invited to gaze on the fixation point and a position of the blind spot of a subject eye of the examinee to the fixation point is obtained in advance in such a fixation state. Subsequently, in order to confirm the fixation state of the examinee during the perimetry, a light stimulation is given to the blind spot at an optional point of time, and if no response to the light stimulation is confirmed (that is, the subject eye does not respond to the light stimulation as long as the subject eye fixates the fixation point even if such stimulation is given to the measured blind spot), it is possible to confirm that the examinee does not watch points excluding the fixation point during the examination.

The perimeter has a jaw stand and a forehead pad in order not to move a position of a face as much as possible during the perimetry, and in some cases, the examination is conducted, fixing a face of the examinee with a headband. Besides, the perimeter has an optical system for observing subject eyes (including a fixation monitoring camera and a monitor), and the examination is conducted, confirming that a reference portion, such as a pupil center of the subj ect eye, is not shifted from a target that show a center position of a screen of a monitor or the like (the fixation point) at a time of start of the examination or during the examination. If shifted, the reference portion of the subject eye is moved and adjusted to the center position by moving the jaw stand in a right-left direction and an up-down direction. By doing so, it is possible to conduct the examination in a state that the subject eye always watches the center of the dome from a predetermined position.

As mentioned before, an important point in order to obtain correct examination results is the examinee always watches the fixation point, and the position of the face of the examinee is fixed at a predetermined position.

### PRIOR ART

### PATENT RELATED DOCUMENT

[Patent related document 1]: Japanese Patent Application Publication No.2008-36297

### DISCLOSURE OF INVENTION

### PROBLEMS TO BE SOLVED BY INVENTION

However, in a conventional perimeter, the rotional state of the face every each examination is not considered at all although the position of the face of the examinee is corrected by adjusting the position of jaw stand in the right-left direction and the up-down direction in advance of the examination, so that it may affect unevenness of the examination results.

In a recent trial, results of perimetry on subject eyes are accumulated every each disease (including normal condition), and proper databases are prepared in an apparatus or through a communication line, such as the Internet, and a change of the visual field of the subject eyes with time is observed in order to use diagnosis of the subject eyes. In such a case, the examination position of the subject eye is shifted every each examination if the face of the examinee rotates every each examination. In such a situation, it is difficult to compare the examination results at the same examination position of the subject eye, and this leads to the unevenness of the examination results and degradation of reliablility.

An object of the invention is to provide the perimeter for conducting perimetry several times, correcting the unevenness of the examination positions due to a rotation of the face of the examinee at the time of perimetry.

### MEANS FOR SOLVING PROBLEMS

A first aspect of the invention is a perimeter (2) having a coordinate system setter (19) that sets a measurement coordinate system (CS) for perimetry on subject eyes (22a), a blind spot detector (13) that detects a position (M1) of a blind spot (22c) of the subject eye, and a perimetry means (19) that measures a visual field of the same subject eye at a first point of time and a subsequent second point of time after the first point of time with the measurement coordinate systems (CS_{F}, CS_{N}) that are set at respective points of time as references, comprising:
a blind spot store that stores a position (M1, (r, *θ*)) of the blind spot that is detected at the first point of time in a memory (15) at the first point of time of perimetry;
a blind spot movement computer (17) that detects the position (M1, (r' , *θ*')) of the blind spot (22c) of the subject eye at the second point of time of perimetry through the blind spot detector (13), and reads the position of the blind spot and the position (M1, (r, *θ*)) of the blind spot at the first point of time that is stored in the memory (15) and computes amount of deviation (α) between both; and
a measurement coordinate corrector (17) that conducts such a correction operation that the measurement coordinate system (CS_{N}) that is set at the second point of time is moved based upon the computed amount of deviation (α) so as to correspond with the measurement coordinate system (CS_{F}) at the first point of time;
   whereby the perimetry means (19) conducts the perimetry based upon the measurement coordinate system (CS_{N}) that corresponds with the measurement coordinate system (CS_{F}) at the first point of time.

A second aspect of the invention is the perimeter, wherein the first point of time is a point of time of first perimetry on the subject eye.

A third aspect of the invention is the perimeter, wherein the position of the blind spot is detected on a polar coordinate (r, *θ*) in which a reference is a center (ZP) of a visual field of the subject eye.

### EFFECTS OF INVENTION

According to the above-mentioned first aspect of the invention, it is possible to conduct the perimetry at the second point of time based upon the measurement coordinate system (CS_{N}) corresponding with one (CS_{F}) at the first point of time in such a way that the amount of deviation (α) of the position of the blind spot is computed every each perimetry, and the measurement coordinate system (CS_{N}) that is set at the second point of time is moved so as to correspond with one (CS_{F}) at the first point of time, and to conduct the perimetry several times after prescribed time, correcting the unevenness of the measurement positions due to the rotation of the face of the examinee at the time of perimetry.

According to the second aspect of the invention, it is possible to always conduct later perimetry based upon the measurement coordinate system (CS_{F}) at the time of the first perimetry, and to easily find a change of the visual field with time.

According to the third aspect of the invention, when detecting the position of the blind spot by a polar coordinate (r, *θ*) with the center of the visual field (ZP) as a reference, a difference of both in the azimuth angle (deviation angle) is the rotational angle (α) to be corrected of the measurement coordinate system, and easy correction operation is possible thereby.

The number in parentheses shows the corresponding element in the drawings for the sake of convenience, accordingly, the descriptions are not restricted and bound by the descriptions on the drawings.

### BRIEF DESCRIPTION OF DRAWINGS

[Fig.1] Fig.1 is a perspective view that shows an example of a perimeter to which the invention is applied.
[Fig.2] Fig.2 is a control block diagram that shows an example of control portions of the perimeter of Fig.1.
[Fig.3] Fig.3 is a typical view that shows a relationship between a face position and a perimetry coordinate at a time of perimetry.
[Fig.4] Fig.4 is a typical view that shows a relationship between the face position and the perimetry coordinate when the face position is rotated in a left direction at the time of perimetry.
[Fig.5] Fig.5 is a typical view that shows a relationship between the face position and the perimetry coordinate when the face position is rotated in a right direction at the time of perimeter.

### PREFERRED EMBODIMENT

An embodiment of the invention is now explained, referring to appended drawings.

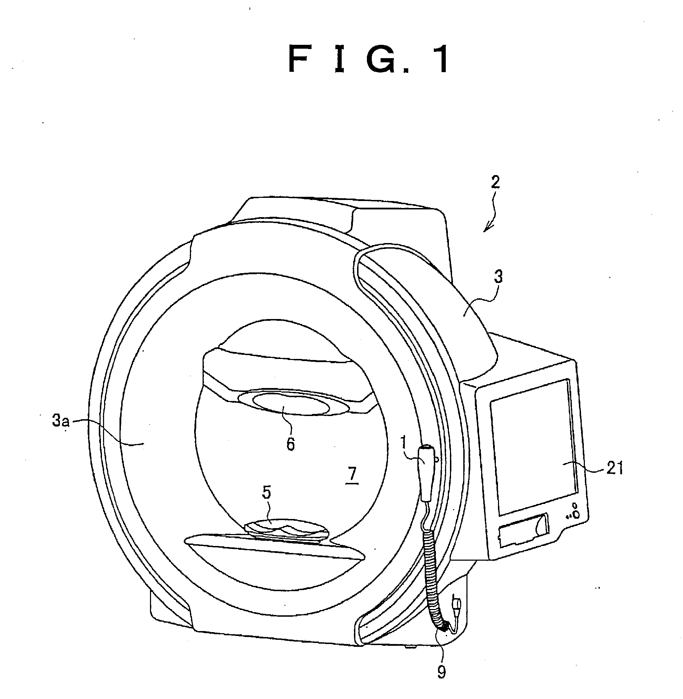

As shown in Fig.1, a perimeter 2 has a main body 3 the whole of which is in the shape of a box, and a jaw stand 5 and a forehead pad 6 are provided at a front face 3a of the main body 3. A response switch 1 is attachably and detachably located on a right side of Fig.1 of the main body 3 through a connection flex 9, and a visual field dome 7 in a semi-spherical shape through which stimuli are presented, is provided at a front hand of the jaw stand 5 and the forehead pad 6, that is, inside the main body 3 on a back side of a paper of Fig.1. The visual field dome 7 is configured so as to project stimulus for perimetry (not shown) at optional positions in the visual field dome 7 through a stimulus presenter 10 that is built in the main body 3.

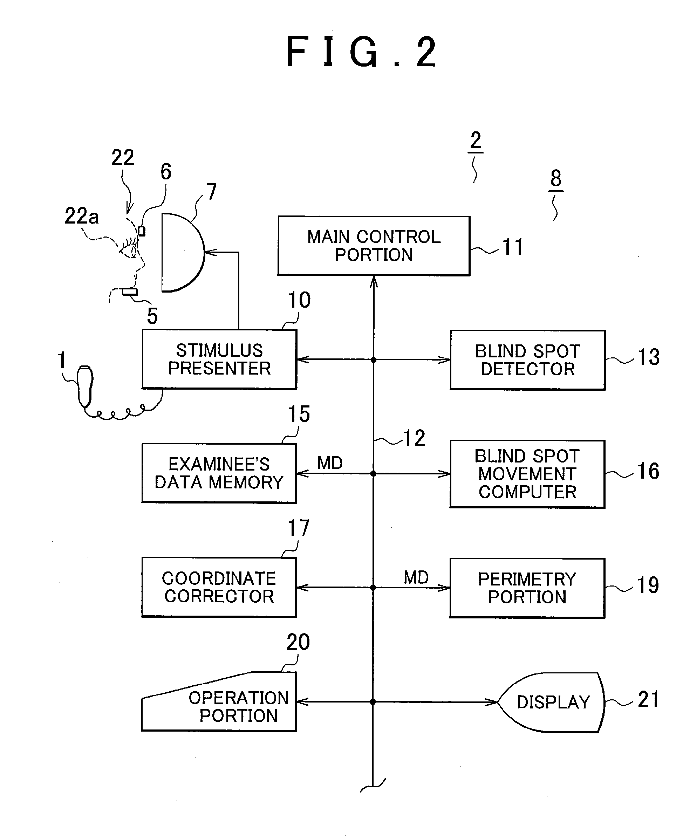

Besides, as shown in Fig.2, a control portion 8 of the perimeter 2 is provided inside the main body 3, and the control portion 8 has a main control portion 11. The before-mentioned stimulus presenter 10, a blind spot detector 13, an examinee's data memory 15, a blind spot movement computer 16, a coordinate corrector 17, a perimetry portion 19, an operation portion 20, such as a keyboard, and a display 21 with the main control portion 11 through a bus line 12. A control block diagram as shown in Fig.2 shows only portions that relate to the invention and does not show the other constitutional portions of the perimeter 2 that are not relate to the invention.

The perimeter 2 has the above-mentioned structure. For a purpose of a perimetry on subject eyes 22a of examinee 22, an examinee is invited to put his (her) jaw on the jaw stand 5 and contact his (her) forehead with the forehead pad 6 as shown in Fig.2 so as to be pressed against such a pad such that the subject eye 22a of a front eye portion of the examinee 22 is located at a predetermined perimetry position.

The front eye portion of the subject eye 22a of the examinee 22 is displayed on the display 21 through a camera (not shown) that is located inside the visual field dome 7, and an operator instructs the examinee 22 to fixate a fixation point that is set inside the visual field dome 7, and adjusts a position of the subject eye 22a so as to be at a front face of the visual field dome 7, that is, at a center of the visual field dome 7 in such a way that the jaw stand 5 and the forehead pad 6 are moved in a right-left direction and an up-down direction in Fig.1 for minor adjustments, watching an image of the front eye portion of the subject eye 22a displayed on the display 21.

In such a state that the positions of the jaw stand 5 and the forehead pad 6 are adjusted so as to position the subject eye 22a at the front face of the visual field dome 7, that is, at the center of the visual field dome 7, and the subject eye 22a of the examinee 22 fixates the fixation point from the center position of the visual field dome 7, the position of the fixation point corresponds with a macula lutea center 22b that is the visual field center of the subject eye 22a.

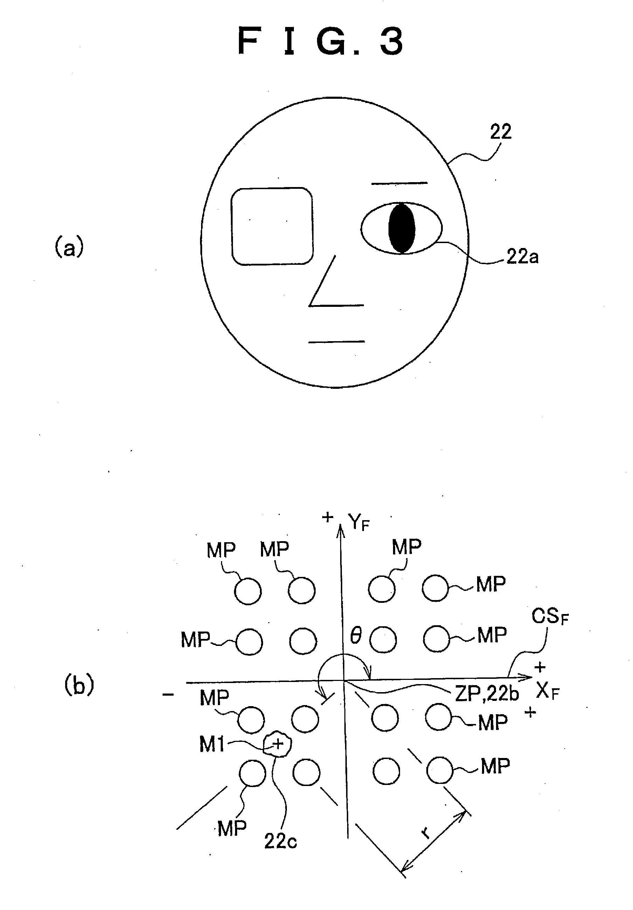

After the fixation point is corresponded with the macula lutea center 22b that is the visual field center of the subject eye 22a through the fixation of the fixation point by the subject eye 22a, the main control portion 11 instructs the blind spot detector 13 to enter an operation of detection of a position M1 of the blind spot (optic disc) 22c. Such a detection operation of the position M1 of the blind spot 22c is conducted based upon a blind spot search program that is built in the blind spot detector 13, but such an action is well-known and its detailed explanation is not mentioned. After obtaining the macular lutea center 22b of the position M1 of the blind spot 22c, that is, the position to the visual field center by searching the blind spot 22c through the blind spot detector 13, the main control portion 11 instructs the perimetry portion 19 to set a measurement coordinate system CS_{F} for perimetry in which a reference (origin ZP) is the fixation point, that is, the visual field center, as shown in Fig.3(b). After setting the measurement coordinate system CS_{F}, the blind spot detector 13 stores a coordinate position of the detected position M1 of the blind spot 22c as measurement data MD of the subject eye 22a of the examinee 22 in the examinee' s data memory 15 together with ID data of the subject eye 22. Preferably, a polar coordinate to the origin ZP (the visual field center or the fixation point) is detected as the coordinate position of the blind spot 22c and such polar coordinate is stored (Of course, a normal rectangular coordinate, such as the coordinate system CS_{F}, may be used). For example, in case of Fig.3(a), the coordinate of the position M1 of the blind spot 22c is (r, *θ*).

When the operator instructs the perimeter 2 to start the perimetry operation on the subject eye through the operation portion 20 in the afore-mentioned state, the main control portion 11 instructs the perimetry portion 19 to measure the visual field of the subject eye. Receiving this instruction, the perimetry portion 19 presents stimuli (not shown) at proper positions inside the visual field dome 7 in order with a well-known method. In such a measurement, stimuli are presented in order at many measurement points MP that are set at appropriate intervals on the measurement coordinate system CS_{F} based upon the origin ZP and the blind spot M1, as shown in Fig.3(b). For convenience of explanation, Fig. 3 (b) shows only sixteen measurement positions MP, but dozens of measurement positions MP are actually set.

When the examinee perceives the stimulus presented on the visual field dome 7 through the subject eye 22a, he (or she) operates the response switch 1. If not perceived, the examinee does not operate the response switch 1. So, the perimetry portion 19 measures the visual field of the subject eye with a well-known method, relating the operational state of the response switch 1 and the position of the stimulus inside the visual field dome 7 at this time.

After thus obtaining a series of the perimetry results regarding the subject eye 22 by the instruction from the main control portion 11, the measurement data MD is stored from the perimetry portion 19 to a memory area attaching ID data corresponding to the subject eye 22a in the examinee's data memory 15 as the measurement data MD of the subject eye 22a of the examinee 22. By doing so, the measurement data MD of the subject eye 22a is stored together with the coordinate (r, *θ*) of the position M1 of the blind spot 22c in which the origin ZP at the time of obtaining the measurement data MD is a reference. The position of the origin ZP is the visual field center of the subject eye 22a that fixates the fixation point, as mentioned before, and corresponds with the position of the macula lutea center 22b of a fundus of the subject eye 22a, as shown in Fig.3(b).

When the second, the third or the n-th perimetry is conducted several months or several years after first perimetry on the subject eye 22, or at constant time intervals after the first perimetry, the operator invites the examinee to put his (her) jaw on the jaw stand 5 and contact his (her) forehead on the forehead pad 6 so as to be pressed, and positions the subject eye 22a at a front face of the visual field dome 7 in such a way that the jaw stand 5 and the forehead pad 6 are moved in the up-down direction and in the right-left direction in Fig.1 for minor adjustment, watching the image of the subject eye 22a displayed on the display 21, similar to the before-mentioned case. Furthermore, in such a state the examinee 22 fixates the fixation point, such a fixation point is set as the visual field center (macula lutea center 22b) of the subject eye 22a.

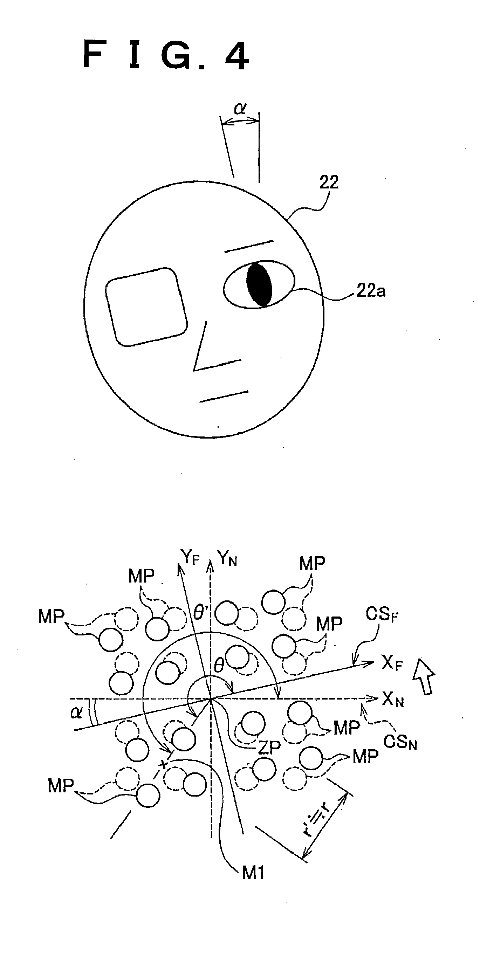

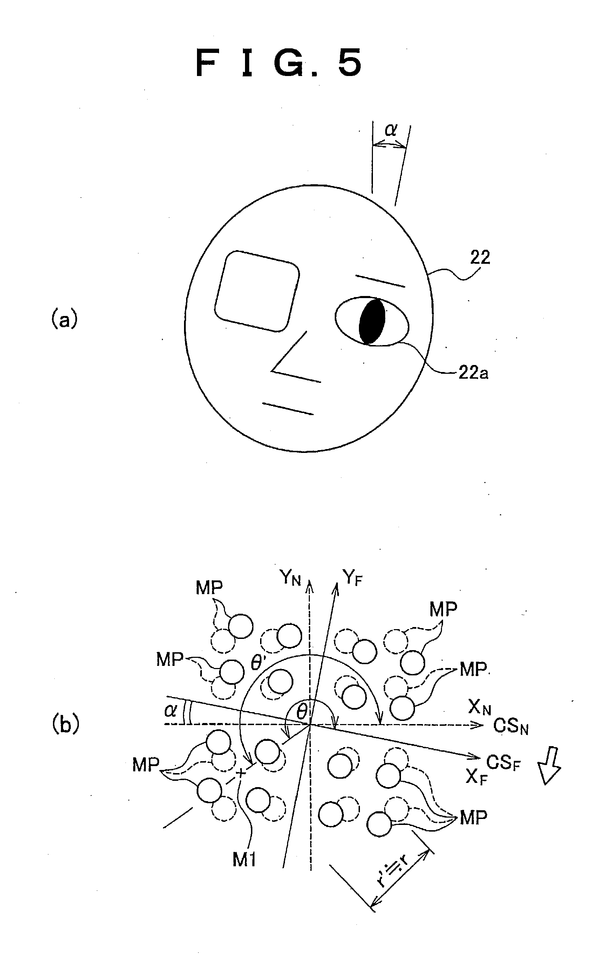

In the afore-mentioned state, the blind spot 22c to the subject eye 22a is detected through the blind spot detector 13, and the measurement coordinate system CS_{N} in which the visual field center is the origin ZP is set. And, the coordinate (r', *θ*') of the blind spot M1 to the visual field center (the macula lutea center 22b, the origin ZP) is obtained by operation. Usually, the macula lutea center 22b that is the visual field center, that is, the position of the origin ZP has hardly an error even after a lapse of prescribed time from the last examination, and is able to be obtained at the position the same as the first examination with high accuracy. But, the position M1 of the blind spot 22c changes since an abutment condition between the examinee and the jaw stand 5 and/or the forehead pad 6 is different each perimetry, and a face (that is, the position of the subject eye 22a) may be inclined as a whole, rotating in a left direction in the figure, as shown in Fig.4(a), or may be inclined as a whole, rotating in a right direction in the figure, as shown in Fig.5(a).

After computing and detecting the coordinate (r', *θ*') of the position M1 of the blind spot 22c through the blind spot detector 13, the blind spot movement computer 16 computes how much the position of the blind spot 22c is moved by rotation with respect to the origin ZP, that is, how much it is shifted from the origin ZP (amount of deviation) in comparison with the first examination at this time of examination through the main control portion 11. In such a case, the blind spot movement computer 16 reads the coordinate (r, *θ*) of the position M1 of the blind spot 22c for the subject eye 22a at the time of the first examination that is stored in the examinee's data memory 15, and compares both azimuth angles *θ* and *θ*' with each other. If the subject eye 22a is rotated angle α in the left direction around the origin ZP in comparison with the first measurement as shown in Fig.4(a), the azimuth angle *θ*' of the coordinate (r' , *θ*') of the measured position M1 of the blind spot 22c is rotated α in the left direction with respect to the azimuth angle *θ* of the position M1 of the blind spot 22c at the time of the first examination.

That is, a relation α=*θ*'- *θ* occurs. This means such a deviation relationship between the measurement coordinate system CS_{N} that is set at the time of this measurement and the firstly set measurement coordinate system CS_{F} that the measurement coordinate system CS_{N} that is set at this time of examination is rotated angle α = *θ* ' - *θ* in the right direction with respect to the firstly set measurement coordinate system CS_{F}, as shown in Fig.4(b).

Then, the main control portion 11 instructs the coordinate corrector 17 to make a revision so as to correspond the measurement coordinate system CS_{N} that is set at the time of this examination and the measurement coordinate system CS_{F} that is set at the first perimetry with each other. Receiving such an instruction, the coordinate corrector 17 rotates the measurement coordinate system CS_{N} that is set at the time of this examination angle α (deviation angle) around the origin ZP in the left direction so that the measurement coordinate system CS_{N} and the measurement coordinate system CS_{F} correspond with each other, as shown in Fig.4(b). By doing so, the measurement point MP that is set on the measurement coordinate system CS_{N} in advance is also rotated angle α together with the rotation of the measurement coordinate system CS_{N} and corresponds with the measurement point MP that is set on the measurement coordinate system CS_{F}.

In the afore-mentioned state, the main control portion 11 conducts the perimetry operation based upon the measurement point MP that is set on the measurement coordinate system CS_{N} similar to the first examination. On this occasion, the measurement point MP that is set on the measurement coordinate system CS_{N} corresponds with the measurement point MP that is set on the measurement coordinate system CS_{F} that is used in the first perimetry operation, and it is possible to conduct this time of perimetry at the positon correctly corresponding with the each measurement point MP in the first perimetry, and to conduct the perimetry, correcting the rotation of the subject eye 22a of the examinee 22.

For this reason, the measurement results in each measurement point MP correctly correspond to ones in each measurement point MP of the first time, and unevenness of the measurement results in each measurement point MP disappears, thereby improving reliability.

In a case where the face of the examinee is rotated angle α in the right direction around the origin ZP at a new perimetry in comparison with the first examination as shown in Fig.5(a), for example, the azimuth angle *θ*' of the coordinate (r' , *θ*') of the measured position M1 of the blind spot 22c is rotated α with respect to the azimuth angle *θ* of the position M1 of the blind spot 22c at the time of first examination.

That is, a relation α=θ' - θ occurs (α< 0 in the right direction.)
This means such a deviation relationship between the measurement coordinate system CS_{N} that is set at this time of examination and the firstly set measurement coordinate system CS_{F} that the measurement coordinate system CS_{N} that is set at this time of examination is rotated angle α = *θ*' - *θ* in the left direction with respect to the firstly set measurement coordinate system CS_{F}, as shown in Fig.5(b).

Then, the main control portion 11 instructs the coordinate corrector 17 to make a revision so as to correspond the measurement coordinate system CS_{N} that is set at this time of examination and the measurement coordinate system CS_{F} that is set at the time of first perimetry with each other. Receiving such an instruction, the coordinate corrector 17 rotates the measurement coordinate system CS_{N} that is set at the time of this examination angle α around the origin ZP in the right direction so that the measurement coordinate system CS_{N} and the measurement coordinate system CS_{F} correspond with each other, as shown in Fig.5(b). By doing so, the measurement point MP that is set on the measurement coordinate system CS_{N} in advance is also rotated angle α in the right direction together with the rotation of the measurement coordinate system CS_{N} and corresponds with the measurement point MP that is set on the measurement coordinate system CS_{F}.

In the afore-mentioned state, the main control portion 11 conducts the perimetry operation based upon the measurement point MP that is set on the measurement coordinate system CS_{N} similar to the first examination. On this occasion, the measurement point MP that is set on the measurement coordinate system CS_{N} corresponds with the measurement point MP that is set on the measurement coordinate system CS_{F} that is used in the first perimetry operation, and it is possible to conduct the perimetry this time at the position correctly corresponding with the each measurement point MP in the first perimetry, and to conduct the perimetry, correcting the rotation of the subject eye 22a of the examinee 22.

For this reason, the measurement results in each measurement point MP correctly correspond to ones in each measurement point MP of the first time, and unevenness of the measurement results in each measurement point MP disappears, thereby improving reliability.

In the before-mentioned embodiment, the blind spot position (r, *θ*) with respect to the origin ZP that is an examination reference point of the measurement coordinate system CS_{F} that is set at the time of the examination is stored in a memory together with the perimetry values in the first perimetry, and the blind spot position (r', *θ'*) that is measured on the measurement coordinate system CS_{N} that is set at the time of the subsequent perimetry on the same subject eye 22a and the blind spot position (r, *θ*) at the time of first perimetry are compared with each other, and the perimetry is conducted this time after moving (including relatively moving) the measurement coordinate system CS_{N} at the later perimetry amount corresponding to the deviation of the blind spot position (such as the angle α). This invention can be applied to the perimetry for two or more times that is conducted for the same eye after a prescribed time, that is, the perimetry that is conducted at a first point of time (not always first) and the subsequent second point of time that are different in time, as well as the case of only first perimetry and the subsequent perimetry.

Besides, the first perimetry (the examination) is not always "the first" one in the examinee's life, but may be the first perimetry wherein the perimetry data having high reliability can be obtained, that is, the first perimetry that is conducted in a normal examination condition. That is, the examinee is inexpected in the perimetry at the first time in his life or at several times of perimetry thereafter, and a situation in which a reliability is decreased, such as a poor fixation, a false-negative reaction and a false-positive reaction, is easy to occur. The data of the perimetry having lower reliability is undesirable as original perimeter data, and preferably, such data is not adopted as the examination data at the first point of time. For this reason, it is possible to deal the first examination on the subject eye 22a with the perimeter, that is, the perimetry of the first point of time with the first point of time when obtaining the perimetry data having a higher reliability. Needless to say, the first perimetry can be dealt with one at the first point of time if the examinee is already used to examination and the examination data having higher reliability is obtained although such an examination is the first one with a corresponding perimeter.

### EXPLANATION OF REFERENCE NUMBERS

- 2: perimeter
- 11: blind spot store (main control portion)
- 13: blind spot detector
- 15: memory (examinee's data memory)
- 16: blind spot movement computer
- 17: coordinate corrector
- 19: coordinate system setter, perimetry means (perimetry portion)
- 22a: subject eye
- 22b: macula lutea center
- 22c: blind spot
- CS, CS_{F}, CS_{N}: measurement coordinate system
- M1: position
- ZP: visual field center(origin)
- α: amount of deviation (angle)

## Claims

1. A perimeter having a coordinate system setter that sets a measurement coordinate system for perimetry on subject eyes, a blind spot detector that detects a position of a blind spot of the subject eye, and a perimetry means that measures a visual field of the same subject eye at a first point of time and a subsequent second point of time with the measurement coordinate systems that are set on respective points of time as references, comprising:
a blind spot store that stores a position of the blind spot that is detected at the first point of time in a memory at the first point of time of perimetry;
a blind spot movement computer that detects the position of the blind spot of the subject eye at the second point of time of perimetry through the blind spot detector, and reads the position of the blind spot and the position of the blind spot at the first point of time that is stored in the memory and computes amount of deviation between both; and
a measurement coordinate corrector that conducts such a correction operation that the measurement coordinate system that is set at the second point of time is moved based upon the computed amount of deviation so as to correspond with the measurement coordinate system at the first point of time;
whereby the perimetrymeans conducts the perimetry based upon the measurement coordinate system that corresponds with one at the first point of time.

2. The perimeter according to claim 1, wherein the first point of time is a point of time of first perimetry on the subject eye.

3. The perimeter according to claim 1, wherein the position of the blind spot is detected on a polar coordinate in which reference is a center of a visual field of the subject eye.
